# EUROPEAN PATENT SPECIFICATION

(11) **EP 2 946 213 B1**
(45) Date of publication and mention of the grant of the patent: **24.05.2023**
(21) Application number: 14700673.8
(22) Date of filing: 16.01.2014
(51) Int. Cl.: G01N 33/68

(54) **PREDICTION OF KIDNEY DISEASE PROGRESSION USING HOMOARGININE AS A BIOMARKER**
VORHERSAGE DES FORTSCHREITENS EINER NIERENERKRANKUNG UNTER VERWENDUNG VON HOMOARGININ ALS BIOMARKER
PRÉDICTION DE LA PROGRESSION D'UNE MALADIE RÉNALE À L'AIDE DE L'HOMOARGININE EN TANT QUE BIOMARQUEUR

(30) Priority: 18.01.2013 EP 13151829
(43) Date of publication of application: 25.11.2015
(73) Proprietor: synlab Services GmbH, 86156 Augsburg (DE); Medizinische Universität Innsbruck, 6020 Innsbruck (AT)
(72) Inventor: DRECHSLER, Christiane, 97078 Würzburg (DE); KOLLERITS, Barbara, A-6020 Innsbruck (AT); MEINITZER, Andreas, A-8010 Graz (AT); PILZ, Stefan, A-8010 Graz (AT); MÄRZ, Winfried, 69493 Hirschberg/Leutershausen (DE); WANNER, Christoph, 97080 Höchberg (DE); KRONENBERG, Florian, A-6020 Innsbruck (AT)
(74) Representative: Benedum, Ulrich Max
(86) International application number: PCT/EP2014/050758
(87) International publication number: WO 2014/111443

(56) References cited:
- EP-A1- 2 505 661
- WO-A1-2011/098519
- WO-A2-02/087508
- MÄRZ WINFRIED ET AL: "Homoarginine, cardiovascular risk, and mortality", CIRCULATION, vol. 122, no. 10, 7 September 2010 (2010-09-07), pages 967-975, XP002625568, LIPPINCOT WILLIAMS AND WILKINS, BALTIMORE, US ISSN: 1524-4539, DOI: 10.1161/CIRCULATIONAHA.109.908988 [retrieved on 2010-08-23]
- DRECHSLER, C., ET AL: "Homoarginine, heart failure, and sudden cardiac death in haemodialysis patients", EUROPEAN JOURNAL OF HEART FAILURE, vol. 13, 2011, pages 852-859, XP002697568,
- MARESCAU B ET AL: "Guanidino compounds in serum and urine of nondialyzed patients with chronic renal insufficiency", METABOLISM, CLINICAL AND EXPERIMENTAL, vol. 46, no. 9, 1 September 1997 (1997-09-01), pages 1024-1031, XP004539068, W.B. SAUNDERS CO., PHILADELPHIA, PA, US ISSN: 0026-0495, DOI: 10.1016/S0026-0495(97)90273-0
- D. FLISER ET AL: "Asymmetric Dimethylarginine and Progression of Chronic Kidney Disease: The Mild to Moderate Kidney Disease Study", JOURNAL OF THE AMERICAN SOCIETY OF NEPHROLOGY, vol. 16, no. 8, 1 August 2005 (2005-08-01) , pages 2456-2461, XP055063858, ISSN: 1046-6673, DOI: 10.1681/ASN.2005020179

## Description

The present invention relates to the field of laboratory diagnostics. Specifically, means and methods for determining the progression of chronic kidney disease (CKD) and/or kidney transplant failure (i.e. kidney allograft loss) including determining the risk of progression of chronic kidney disease (CKD) and/or the risk of kidney transplant failure (i.e. kidney allograft loss) based on the analysis of homoarginine levels are disclosed.

An aim of modern medicine is to provide personalized or individualized treatment regimens. Those are treatment regimens which take into account a patient's individual needs or risks. Individualized treatment regimens offer benefits for the individual patient as well as for society as a whole. For the individual patient personalized treatment avoids excessive therapy while ensuring that necessary measures are taken. As every therapy may cause undesired harmful side effects, the avoidance of unnecessary therapies saves the patient from potentially harmful side effects. On the other hand, the identification of patients with special needs ensures that these individuals receive the appropriate treatment. For the health system as a whole, the avoidance of unnecessary therapies allows for a more economic use of resources.

Individualized treatment regimens require appropriate diagnostic tools in order to separate those patients who benefit from certain therapeutic measures from the patients who do not. Therefore, the development of individualized treatment regimens critically depends on the development of novel diagnostic tools and procedures. Because the prevention of future disease is often more effective than the therapy of already existing disease, diagnostic tools and methods for risk stratification with respect to future diseases are especially desirable.

Chronic kidney disease (CKD) represents a major public health problem with an increasing prevalence as well as an increase in the incidence rate of end-stage renal disease (Coresh et al., 2007, JAMA 298: 2038-2047; US Renal System. USRDS 2008 Annual Data Report. Bethesda, MD: National Institutes of Health, National Institute of Diabetes and Digestive and Kidney Diseases, 2008). The costs of treatment put an enormous burden on health care resources since renal replacement therapy represents one of the most expensive chronic therapies. Importantly, CKD per se has been shown to be a strong risk factor for cardiovascular morbidity and mortality (Go et al., 2004, N Engl J Med 351: 1296-1305). Patients with a moderately impaired kidney function already have a high risk to develop cardiovascular complications (Anavekar et al., 2004, N Engl J Med 351: 1285-1295). Cardiovascular risk further increases with the decline in kidney function, and the majority of CKD patients die from cardiac and vascular events before reaching end-stage renal disease.

Kidney transplantation represents the most favorable form of renal replacement therapy. Mortality significantly improved in patients receiving a kidney transplant as compared to those remaining on dialysis. However, cardiovascular disease still represents the major cause of death in kidney transplant recipients, followed by infectious complications. Both contribute to renal function decline and potential graft loss. Strategies are therefore needed to improve cardiovascular and infectious morbidity and mortality in kidney transplant recipients.

Homoarginine is a cationic amino acid, which is derived from lysine and mainly synthesized in the kidney by transaminidation of its precursor (Ryan et al., 1969, Arch Biochem Biophys 131: 521-526; Ryan and Wells, 1964, Science 144: 1122-1127). Studies have shown that homoarginine serves as a precursor of nitric oxide (NO) by increasing the intracellular concentration of L-arginine, which is the main substrate for NO synthase (Hrabak et al., 1994, Biochem Biophys Res Commun 198: 206-212; Knowles et al., 1989, Proc Natl Acad SciU S A 86: 5159-5162; Valtonen et al., 2008, Circ J 72: 1879-1884; Yang and Ming, 2006, Curr Hypertens Rep 8: 54-59).

Thus, homoarginine may increase the availability of NO and impede or ameliorate endothelial dysfunction, which is crucial to prevent progression of CKD. In a clinical study, homoarginine was inversely correlated to ICAM-1 and VCAM-1 as markers of impaired endothelial function (Maerz et al, 2010, Circulation 122: 967-975). This hypothesis is, however, not completely settled since substrate competition between homoarginine and arginine may even decrease catalytic efficiency of NO synthase (Moali et al., 1998, Biochemistry 37: 10453-10460). The level of homoarginine has been found to be significantly decreased in the serum of non-dialyzed chronic renal insufficiency patients (Marescau et al, 1997, Metabolism - Clinical and Experimental 46:1024-1031).

Furthermore, an inverse association with inflammation has been demonstrated (Maerz et al, 2010, Circulation 122:967-975; Drechsler et al., 2011, Eur J Heart Fail 13: 852-859). Hypertension and diabetes mellitus are known to play a major role for the progression of chronic kidney disease (CKD). Interestingly, studies found that administration of L-homoarginine increased urinary excretion of nitrate as the degradation product of NO, and reduced blood pressure in salt-sensitive hypertensive rats (Chen and Sanders, 1993, Hypertension 22: 812-818).

Prevention of disease progression and associated complications therefore is highly important, requiring the knowledge of risk factors and appropriate treatment. Consequently, the problem underlying the present invention could be seen in the identification of additional markers that allow for a risk stratification of patients with respect to acute renal events and/or chronic kidney disease (CKD), including end-stage renal disease and kidney transplant failure (i.e. kidney allograft loss). The scope of the invention is defined by the appended claims. Embodiments and descriptions no longer falling under the scope of the appended claims are considered merely as examples suitable for understanding the invention.

In the context of the present invention, it has surprisingly been found that the level of homoarginine in a patient's sample strongly correlates with the progression of chronic kidney disease (CKD), including end-stage disease (i.e. kidney failure), as well as with the rejection of kidney transplants.

The present invention relates to a method for determining the progression of chronic kidney disease (CKD) and/or kidney transplant failure in a patient, wherein the method comprises the steps of
a) determining the amount of homoarginine in a sample of the patient; and
b) comparing the determined amount of homoarginine with a reference amount, whereby the progression of chronic kidney disease and/or kidney transplant failure is determined.

The term "determining the progression of chronic kidney disease (CKD)" as used herein generally refers to the assessment of kidney function in a patient including the assessment of end-stage disease. In particular, it refers to the assessment of well a patient's kidney functions and/or how severe a patient's kidney disease is. Standard parameters that are routinely used for diagnosing kidney disease in a patient are well known in the art and familiar to the skilled person. In the present context, the term "end-stage disease" is equivalently used to kidney and/or renal failure. The term "determining kidney transplant failure" as used herein generally refers to the assessment of kidney transplant rejection including the risk that a kidney transplant that has before been implanted into a patient in need thereof will be rejected and/or is not well accepted by the patient's organism. In the context of the present invention, the term "kidney transplant failure" also refers to and is equally used for kidney allograft loss. Therefore, preferably, the method of the invention also includes assessing the risk of chronic kidney disease progression and/or assessing the risk of kidney transplant failure (i.e. kidney allograft loss).

The method of the invention further comprises determining the risk of chronic kidney disease (CKD) progression and/or the risk of kidney transplant failure in a patient.

Preferably, the progression of chronic kidney disease (CKD) in a patient according to the present invention also includes end-stage renal disease.

In the context of the present invention, the progression of chronic kidney disease (CKD) is generally determined by comparing the amount of homoarginine from a patient sample with a reference amount. Preferably, this reference amount is derived from a sample of a healthy patient.

The term "comparing" as used herein encompasses comparing the amount of homoarginine comprised by the sample to be analysed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amounts determined in step a) and the reference amount of the method of the present invention, it is possible to predict the risk of the subject of suffering of one or more of the complications referred to herein. Therefore, the reference amount is to be chosen so that either a difference or a similarity in the compared amounts allows identifying those patients with an increased progression in chronic kidney disease (CKD) or with an increased risk of kidney transplant failure.

In the context of the present invention, the term "reference amount" may refer to a reference that may be (i) derived from a patient known to be at increased risk of chronic kidney disease (CKD), or (ii) it may be derived from a patient known not to be at increased risk of chronic kidney disease (CKD), including, for example, a healthy patient. Preferably, the reference amount is determined on the basis of an averaged median amount obtained from a group of patients meeting the criteria either of (i) or of (ii), described above. Moreover, the reference amount may define a threshold amount, whereby an amount smaller than the threshold shall be indicative for a subject which is at increased risk of mortality. The reference amount applicable for an individual subject may vary depending on various physiological parameters such as age, gender, or subpopulation, as well as on the means used for the determination of the amino acid referred to herein. A suitable reference amount may be determined by the method of the present invention from a reference sample to be analysed together, i.e. simultaneously or subsequently, with the test sample. A preferred reference amount serving as a threshold may be derived from the lower limit of normal (LLN), i.e. the lower limit of the physiological amount to be found in samples from a population of subjects not being at increased risk of mortality. The LLN for a given population of subjects can be determined by various well known techniques. A suitable technique may be to determine the median or average of the population for the amino acid amounts to be determined in the method of the present invention.

A decreased amount of homoarginine in comparison to the reference amount indicates the progression of chronic kidney disease (CKD) and a decreased amount of homoarginine in comparison to the reference amount indicates kidney transplant failure.

In the context of the present invention, the progression of chronic kidney disease (CKD) can also be determined by comparing amounts of homoarginine from more than one sample of a patient, characterized in that these samples have been taken from the patient at different time points. Here, any difference between the determined amounts of homoarginine indicates a progression of the patient's disease status, if the amount of homoarginine is determined to be lower in the sample taken from the sample at a later time point as compared to the amount of homoarginine as determined in the sample taken at an earlier time point. Alternatively, vice versa, if the determined amount of homoarginine is determined to be increased in the sample taken from the patient at a later time point in comparison to the sample taken from the patient at an earlier time point, this difference would indicate an improvement of the patient's health status. Accordingly, the term "progression of chronic kidney disease (CKD)" as used herein can also mean the assessment of an improvement of the disease status.

The term "determining the risk of progression of chronic kidney disease" or "determining the risk of kidney transplant failure" as used herein refers to assessing the probability according to which a subject will suffer from a progressed disease status within a certain time window, i.e. the predictive window. In accordance with the present invention, the predictive window, preferably, is within 1 year, 2 years, 4 years, 6 years, 8 years, 10 years or more after the chronic kidney disease has been diagnosed or after the kidney transplant has been implanted into the patient. Most preferably, the predictive window is within 4 years, 5 years or 6 years. However, as will be understood by those skilled in the art, such an assessment is usually not intended to be correct for 100 % of the subjects to be investigated. The term, however, requires that prediction can be made for a statistically significant portion of subjects in a proper and correct manner. Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Preferably, the probability envisaged by the present invention allows that the prediction of an increased, normal or decreased risk will be correct for at least 60 %, at least 70 %, at least 80 %, or at least 90 % of the subjects of a given cohort or population. The term, preferably, relates to determining whether or not there is an increased risk of progression of disease or kidney transplant failure as compared to the average risk of disease progression in a population of subjects rather than giving a precise probability for the said risk.

Accordingly in the present invention, a decreased amount of homoarginine in comparison to the reference amount indicates an increased risk of progression of chronic kidney disease (CKD) and/or an increased risk of kidney transplant failure.

The method of the present invention is an in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or step (b) may be assisted, either in total or in part, by automation, e.g., by a suitable robotic and sensory equipment for the determination in step (a) or a computer-implemented comparison in step (b).

The term "patient", preferably, refers to a mammal, more preferably to a human. In a preferred embodiment of the present invention, the patient is healthy with respect to diseases that increase the risk of renal failure. Said diseases are, preferably, hypertension, type 1 diabetes, type 2 diabetes and/or cardiovascular diseases. In a further preferred embodiment of the present invention, the patient suffers from chronic kidney disease and/or has received a kidney transplant. In yet another preferred embodiment of the present invention, the patient is in need of receiving a kidney transplant.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which produce the marker referred to herein.

In a preferred embodiment, the sample of the patient is a blood sample. More preferably, the sample is a plasma sample.

The term "homoarginine" refers to a chemical compound which is described in formula (I) below. Homoarginine is, preferably, L-homoarginine.

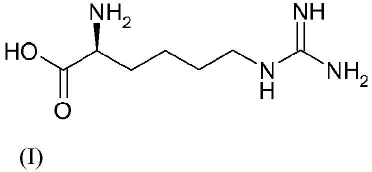

Determining the amount of homoarginine relates to measuring the amount or concentration, preferably semi-quantitatively or quantitatively. Measuring can be done directly or indirectly. Direct measuring relates to measuring the amount or concentration of homoarginine based on a signal which is obtained from the amino acid itself and the intensity of which directly correlates with the number of molecules of the amino acid present in the sample. Such a signal - sometimes referred to herein as intensity signal - may be obtained, e.g., by measuring an intensity value of a specific physical or chemical property of the amino acid. Indirect measuring includes measuring of a signal obtained from a secondary component (i.e. a component not being the amino acid itself) or a biological read out system, e.g., measurable cellular responses, ligands, labels, or enzymatic reaction products. Furthermore, the use of immunoassays for the determination of the marker of the present invention is preferred.

In accordance with the present invention, determining the amount of homoarginine can be achieved by all known means for determining the amount of an amino acid in a sample. Said means, preferably, comprise chromatographic methods detections or methods based on the formation of coloured reaction products.

Especially preferred is the use of chromatographic methods for determining the amount of homoarginine. Most preferred are high performance liquid chromatography (HPLC) and gas chromatography (GC). Gas chromatography and liquid chromatography are, preferably, coupled to mass spectrometry (GC-MS, HPLC-MS) for the identification of the amino acid. These methods are well known to the person skilled in the art. Moreover, most preferably used for determining the amount of homoarginine is high performance liquid chromatography (HPLC) coupled with fluorescence detection, whereby homoarginine and an internal standard from the biological sample are extracted via ion-exchange-solid phase extraction (SPE). Subsequently, the extract is converted into a fluorescent derivative using the reagents ortho-phthalaldehyde and mercaptan (e.g. 2-Mercaptoethanol, 3-Mercaptopropionic acid). The fluorescent derivatives are separated via HPLC and quantitatively determined using fluorescence detection (Meyer, J et al, 1997, Anal Biochem, 247:11-6). The person skilled in the art is well aware of various modifications of this method (WO 2006/128419).

In a preferred embodiment, the amount of homoarginine in step a) is determined by means of reverse-phase high-performance liquid chromatography (HPLC).

Further preferred chromatographic separation methods for determining the amount of homoarginine include capillary electrophoresis coupled with fluorescence detection, gas chromatography tandem mass spectrometry subsequently after extraction and derivatization (as methylester tri(N-pentafluoropropionyl) derivative), or liquid chromatography-tandem mass spectrometry (HPLC-MS/MS) involving the use of two mass spectrometers, in tandem, as the detector for an HPLC.

It is further preferred that determining the amount of homoarginine comprises the step of measuring a specific intensity signal obtainable from homoarginine in the sample.

Determining the amount of homoarginine, preferably, comprises the steps of (a) contacting homoarginine with a specific ligand, (b) optionally removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to homoarginine. Preferred ligands include antibodies, nucleic acids, peptides or polypeptides such as receptors or binding partners for homoarginine and fragments thereof comprising the binding domains for homoarginine. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to homoarginine. Specific binding according to the present invention means that the ligand or agent should not bind substantially to ("cross-react" with) another amino acid, peptide, polypeptide or substance present in the sample to be analyzed. Preferably, the specifically bound homoarginine should be bound with at least 3 times higher, more preferably at least 10 times higher and even more preferably at least 50 times higher affinity than any other relevant substance. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semiquantitative or quantitative. Suitable methods are described in the following.

First, binding of a ligand may be measured directly, e.g. by NMR or surface plasmon resonance.

Second, the ligand may exhibit enzymatic properties itself and the "ligand/peptide or polypeptide" complex or the ligand which was bound to homoarginine, respectively, may be contacted with a suitable substrate allowing detection by the generation of an intensity signal. For measurement of enzymatic reaction products, preferably the amount of substrate is saturating. The substrate may also be labelled with a detectable label prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand. Labelling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star^{™} (Amersham Biosciences), ECF^{™} (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I ³²P, ³³P and the like. A radioactive label can be detected by any method known and appropriate, e.g. a light-sensitive film or a phosphor imager. Suitable measurement methods according to the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The amount of homoarginine may be, also preferably, determined as follows: (a) contacting a solid support comprising a ligand for the homoarginine as specified above with a sample comprising homoarginine and (b) measuring the amount of homoarginine which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

A metabolic precursor of homoarginine is lysine. Lysine is an α-amino acid with the chemical formula HO2CCH(NH2)(CH2)4NH2. It is an essential amino acid, as it is not synthesized in animals, hence it must be ingested as lysine or lysine-containing proteins. In plants and bacteria, it is synthesized from aspartic acid (aspartate). Lysine is a base. The ε-amino group often participates in hydrogen bonding and as a general base in catalysis. Common posttranslational modifications include methylation of the ε-amino group, giving methyl-, dimethyl-, and trimethyllysine. The latter occurs in calmodulin. Other posttranslational modifications at lysine residues include acetylation and ubiquitination. Collagen contains hydroxylysine which is derived from lysine by lysyl hydroxylase. O-Glycosylation of lysine residues in the endoplasmic reticulum or Golgi apparatus is used to mark certain proteins for secretion from the cell. Lysine is metabolised in mammals to give acetyl-CoA, via an initial transamination with α-ketoglutarate. The bacterial degradation of lysine yields cadaverine by decarboxylation. Allysine is a derivative of lysine, used in the production of elastin and collagen. It is produced by the actions of the enzyme lysyl oxidase on lysine in the extracellular matrix and is essential in the crosslink formation that stabilizes collagen and elastin. L-Lysine is a necessary building block for all proteins in the body. L-Lysine plays a major role in calcium absorption; building muscle protein; recovering from surgery or sports injuries; and the body's production of hormones, enzymes, and antibodies. Lysine can be modified through acetylation, methylation, ubiquitination, sumoylation, neddylation, biotinylation, and carboxylation which tends to modify the function of the protein of which the modified lysine residue(s) are a part.

Preferably, an amount of homoarginine above a certain level indicates a low risk of progression of chronic kidney disease (CKD) and/or kidney transplant failure, while an amount of homoarginine below this level indicates an increased risk of progression of chronic kidney disease and/or kidney transplant failure. This certain level is a continuous parameter, i.e. a parameter which can vary from different patients and/or from different disease circumstances. In general, an amount of homoarginine determined to be below about 2.5 µM, preferably below about 2.0 µM, more preferably below about 1.5 µM, and most preferably below about 1.0 µM indicates an increased risk of progression of chronic kidney disease (CKD) and/or an increased risk of kidney transplant failure.

Here, the term "about" is meant to indicate +/- 30 % of the indicated amount, preferably +/-20 % of the indicated amount, more preferably +/- 10 % of the indicated amount, even more preferably +/- 5 % of the indicated amount, and most preferably +/- 1 % of the indicated amount.

In the context of the present invention, it has surprisingly been found that the lower the amount of homoarginine is in comparison to the reference amount, the higher is the progression of chronic kidney disease (CKD) and/or the risk of kidney transplant failure. Here, an almost linear correlation between these parameters has been determined by the Examples provided herein.

Accordingly, in a preferred embodiment, the progression of chronic kidney disease (CKD) and/or the risk of kidney transplant failure is the higher, the lower the amount of homoarginine is in the patient's sample.

In another preferred embodiment, the decreased amount of homoarginine correlates with a decreased renal function. Preferably, the decreased renal function correlates with a decreased glomerular filtration rate (GFR) and/or an increased level of serum creatinine in the patient.

Both the glomerular filtration rate (GFR) and the level of serum creatinine are standard clinical parameter for determining the renal function in a patient. Methods of how to determine these parameters are routine work for the skilled person and well established in the art (Schnabel et al., 2010, Eur Heart J 31, 2024-3031). That is, preferably, renal function is defined by the patient's blood concentration of serum creatinine. Serum creatinine is the most commonly used indicator of renal function. The concentration of serum creatinine can be measured in a blood sample by standard methods, such as the Jaffe routine method. The concentration of serum creatinine is preferably calculated in mg/dl, but may be indicated by any other suitable concentration format, such as, for example, pmol/dl.

Renal function is defined by the patient's blood concentration of cystatin C. Cystatin C, also referred to in the arte as cystatin 3 (and formerly known as gamma trace, post-gamma-globulin or neuroendocrine basic polypeptide) is a protein encoded by the CST3 gene. Cystatin C is a routinely used biomarker of kidney function of a patient. The concentration of serum cystatin C can be defined by standard methods known in the art and is preferably calculated and indicated in mg/dl.

Advantageously, the present invention provides a reliable biomarker for determining the risk of chronic kidney disease progression and/or kidney transplant failure. The identification of high risk patients allows for a closer monitoring of this group so that preventive treatments can be administered to those patients with the greatest need. Moreover, homoarginine increases the availability of nitric oxide and is probably positively related to endothelial function. This fact taken together with the finding of the study underlying the present invention that low amounts of homoarginine correlate with progression of chronic kidney disease, moreover, suggests specific preventive measures: patients with low amounts of homoarginine should receive therapies that aim at increasing homoarginine and/or NO-levels and at supporting renal function. Another finding of the study is the association of homoarginine with kidney transplant failure in that low serum homoarginine levels are identified as a novel risk factor for the rejection of kidney transplants. Thus, the present invention contributes to the development of individualized treatment regimens.

It is to be understood that the definitions and explanations of the methods, measurements, and terms made above apply mutatis mutandis for all aspects described in this specification in the following except as otherwise indicated.

The present invention takes advantage of further certain markers. The term "marker" is known to the person skilled in the art. In particular, markers are gene expression products which are differentially expressed, i.e. up regulated or down regulated in presence or absence of a certain condition, disease, or complication. Usually, a marker is defined as a nucleic acid (including mRNA), a protein, peptide, or small molecule compound. The amount of a suitable marker can indicate the presence or absence of the condition, disease, or complication, and thus allow diagnosis.

In the context of the present invention, these markers preferably relate to markers of a patient's renal function, including, but not limited to, the glomerular filtration rate (GFR), the plasma level of serum creatinine and the plasma level of cystatin C in the patient.

The link between low amounts of homoarginine and a progression of chronic kidney disease and kidney transplant failure discovered in the study underlying the present invention allows for the identification of those patients who have an increased risk of kidney failure due to a lack of homoarginine. Hence, these patients should receive additional homoarginine to decrease said risk.

The present disclosure further relates to homoarginine for use in a method of treatment and/or prophylaxis of chronic kidney disease (CKD) including end-stage renal disease in a patient.

In a further aspect, the present invention relates to homoarginine for use as a marker in kidney transplant failure.

The present disclosure further relates to homoarginine for use in a method of prevention and/or treatment of kidney transplant failure in a patient.

Homoarginine can be used in a method for treating and/or preventing the progression of chronic kidney disease (CKD) and/or minimizing the risk of kidney transplant failure as described herein in a therapeutically effective dose.

A therapeutically effective dose refers to an amount of the pharmaceutically active compound to be used in a pharmaceutical composition which prevents, ameliorates or treats the symptoms accompanying a disease or condition referred to in this specification. Therapeutic efficacy and toxicity of the compound can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and other clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment.

As used herein, an effective amount of homoarginine is a dosage large enough to produce the desired therapeutic effect to reduce the risk of chronic kidney disease (CDK), end-stage renal failure and/or rejection of a kidney transplant. An effective amount is not, however, a dosage so large as to cause adverse side effects. Generally, an effective amount may vary with the patient's age, condition, weight and sex, as well as the extent of the condition being treated, and can be determined by one of skill in the art. The dosage may be adjusted by the individual practitioner in the event of any complication.

The present disclosure relates to a device for determining the progression of chronic kidney disease (CKD) and/or kidney transplant failure in a patient comprising
a) an analysing unit for determining the amount of homoarginine or its metabolic precursor in a sample of the patient; and
b) an evaluation unit for comparing the determined amount with a reference amount, wherein the unit allows for the evaluation of the progression of chronic kidney disease (CKD) and/or kidney transplant failure.

The term "device" as used herein relates to a system of means comprising at least the aforementioned means operatively linked to each other as to practise the method of the present invention. Preferred means for determining the amounts of the markers of the present invention, and means for carrying out the comparison are disclosed above in connection with the method of the invention. How to link the means in an operating manner will depend on the type of means being included into the device. For example, where an analysis unit for automatically determining the amount of the amino acid of the present invention is applied, the data obtained by said automatically operating analysis unit can be processed by, e.g., a computer as evaluation unit in order to obtain the desired results. Preferably, the means are comprised in a single device in such a case.

Said device, preferably, includes an analysing unit for the measurement of the amount of homoarginine in an applied sample and an evaluation unit for processing the resulting data. Preferably, the evaluation unit comprises a database with the stored reference amounts and a computer program code which when tangibly embedded on a computer carries out the comparison of the determined amounts and the reference amounts stored in the database. More preferably, the evaluation unit comprises a further computer program code which allocates the result of the comparison to a risk prediction. In such a case, it is, also preferably, envisaged that the evaluation unit comprises a further database wherein the reference amounts are allocated to the risks.

Alternatively, where means such as test stripes are used for determining the amount of homoarginine, the evaluation unit may comprise control stripes or tables allocating the determined amount to a reference amount. The test stripes are, preferably, coupled to ligands which specifically bind to homoarginine. The strip or device, preferably, comprises means for detection of the binding of said homoarginine to said ligands. Preferred means for detection are disclosed in connection with embodiments relating to the method of the invention above. In such a case, the analysis unit and the evaluation unit are operatively linked in that the user of the system brings together the result of the determination of the amount and the diagnostic or prognostic value thereof due to the instructions and interpretations given in a manual. The analysis unit and the evaluation unit may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of raw data which need interpretation by the clinician. Preferably, the output of the device is, however, processed, i.e. evaluated, raw data the interpretation of which does not require a clinician. Further preferred devices comprise the analyzing units/devices (e.g., biosensors, arrays, solid supports coupled to ligands specifically recognizing homoarginine, Plasmon surface resonance devices, NMR spectrometers, mass-spectrometers etc.) or evaluation units/devices referred to above in accordance with the method of the invention.

The present disclosure relates to a kit for determining the progression of chronic kidney disease (CKD) and/or kidney transplant failure in a patient comprising
a) an analysing agent for determining the amount of homoarginine or its metabolic precursor in a sample of the patient; and
b) an evaluation unit for comparing the amount determined by the analysing agent with a reference amount, wherein the unit allowing for the evaluation of the progression of chronic kidney disease (CKD) and/or kidney transplant failure.

The term "kit" as used herein refers to a collection of the aforementioned components of which may or may not be packaged together. The components of the kit may be comprised by separate vials (i.e. as a kit of separate parts) or provided in a single vial. Moreover, it is to be understood that the kit can be used for practising the methods referred to herein above. It is, preferably, envisaged that all components are provided in a ready-to-use manner for practising the methods referred to above. Further, the kit preferably contains instructions for carrying out the said methods. The instructions can be provided by a user's manual in paper- or electronic form. For example, the manual may comprise instructions for interpreting the results obtained when carrying out the aforementioned methods using the kit of the present invention. The kit shall comprise an analyzing agent. This agent is capable of specifically recognizing homoarginine in a sample of the subject. Moreover, the said agent(s) shall upon binding to homoarginine, preferably, be capable of generating a detectable signal, the intensity of which correlates to the amount of homoarginine present in the sample. Dependent on the type of signal which is generated, methods for detection of the signal can be applied which are well known in the art. Analyzing agents which are preferably used for the kit include antibodies or aptamers. The analyzing agent may be present on a test stripe as described elsewhere herein. The amount homoarginine thus detected can than be further evaluated in the evaluation unit. Preferred evaluation units to be used for the kit include those referred to elsewhere herein.

It is to be understood that the definitions and explanations of the methods, measurements, and terms made above apply mutatis mutandis for all aspects described in this specification in the following except as otherwise indicated.

The device and/or the kit is defined by any of the the embodiments as described herein.

The following example is only intended to illustrate the present invention and shall not limit the scope of the invention in any way.

### EXAMPLES

### Example 1

### MATERIAL and METHODS

**Baseline investigation.** The methodology of the MMKD Study has previously been reported in detail (Becker et al., 2005, J Am Soc Nephrol 16: 1091-1098; Kollerits et al., 2007, Kidney Int 71:1279-1286). Briefly, the MMKD Study is a prospective cohort study including 227 white patients aged between 18 and 65 years with nondiabetic CKD and various degrees of kidney impairment. The patients were recruited from 8 nephrology departments in Germany, Austria, and South Tyrol (Italy) as previously described (Kronenberg et al., 2000, J Am Soc Nephrol 11: 105-115). The patients had stable kidney function for at least 3 months before inclusion into the study. Exclusion criteria were serum creatinine > 6 mg/dL (531 mol/L), diabetes mellitus of any type, malignancy, liver, thyroid, or infectious diseases, nephrotic syndrome (defined as daily proteinuria >3.5g/1.73m2), organ transplant, immunosuppressive treatment, allergy to ionic contrast media, treatment with fish oil or erythropoietin and pregnancy.

To avoid interobserver differences, all patients were recruited by a single investigator who visited all participating centers. Information on age, gender, smoking habits, comorbidities and antihypertensive treatment at baseline was recorded by patient interview and confirmed by checking patient records. A clinical examination completed the procedure. Body mass index (BMI) was calculated as weight (kg) divided by height (m) squared. Blood pressure was measured in sitting position. Hypertension was defined as BP >140/90 mmHg and/or the use of antihypertensive medication.

**Ethics statement.** The study was approved by the Institutional Ethic Committee of the University of Innsbruck, and all participants gave their informed consent before inclusion in the study.

**Prospective follow-up and outcome assessment.** Patients were followed prospectively until the primary study endpoint or the end of the follow-up period was reached. The primary endpoint was defined as doubling of baseline serum creatinine and/or terminal renal failure necessitating renal replacement therapy. A total of 177 (78 %) patients from the baseline cohort could be followed prospectively over a period of up to 84 months. Patients received regular follow-up care in the outpatient ward. Patients who were lost to follow-up (n=50) had at baseline a significantly better renal function than patients who were not lost for follow-up (i.e., a higher mean GFR [91±44 versus 64±39 ml/min per 1.73 m2; P <0.01]). However, both groups did not differ significantly with respect to age and gender. Patients who were lost to follow-up had moved away or were not referred by their physicians for follow-up visits in the renal units.

**Homoarginine and GFR measurement.** Homoarginine was measured in plasma samples taken at baseline and stored at -80 °C, using a reverse-phase HPLC method (Meinitzer et al., 2007, Clin Chim Acta 384: 141-148; Teelink et al., 2002, Anal Biochem 303: 131-137). Within-day coefficients of variation (CV) were 4.7 % (for a control sample with 1.21 µmol/L) and 2.2% (3.53 µmol/L), and between-day CV were 7.9 % (1.25 µmol/L) and 6.8 % (3.66 µmol/L), respectively. GFR was assessed in all patients using the iohexol clearance technique as described in detail elsewhere (Bostom et al., 2002, J Am Soc Nephrol 13: 2140-2144).

**Statistical analysis.** Continuous variables were compared between various groups using unpaired t tests or the nonparametric Wilcoxon rank sum test in case of non-normally distributed variables. Continuous variables across the stages of CKD were analyzed using one-way ANOVA or Kruskal-Wallis test where appropriate. Dichotomized variables were compared using Pearson χ²-test. We explored the correlation of homoarginine with other parameters using Spearman correlation analysis. Cox regression analyses was applied to calculate hazard ratios (HRs) and corresponding 95 % confidence intervals per one standard deviation change of variables for the progression to renal endpoints adjusted for age, sex, and other risk predictors of disease progression. A two-sided p-value <0.05 was considered statistically significant. Analyses were performed using SPSS for Windows version 18.0.

### RESULTS

**Patient characteristics.** Of all 227 non-nephrotic patients included into the MMKD study, 182 patients had a homoarginine measurement at baseline. Characteristics of patients with and without homoarginine values were not significantly different. The mean homoarginine concentration was 2.57±1.09 µmol/L. The baseline clinical characteristics and laboratory data of these patients with CKD are reported in Table 1. Overall, patients had a mean age of 46±13 years and 67% were male. Of the 182 patients, 59 patients had CKD stage 1 with a GFR ≥90ml/min, 35 patients had stage 2 with a GFR ≥60-89ml/min, 51 patients had stage 3 with a GFR ≥30-59ml/min and 37 patients had CKD stage 4 or 5 with a GFR <30ml/min. Homoarginine concentrations were incrementally lower at lower levels of GFR with a mean concentrations of 2.90±1.02 µmol/L (stage 1), 2.64±1.06 µmol/L (stage 2), 2.52±1.24 µmol/L (in stage 3) and 2.05±0.78 (stage 4-5), respectively. The differences in mean homoarginine concentrations across the stages of CKD were highly significant (p=0.002). By correlation analyses, homoarginine concentrations were significantly related to GFR (Spearman correlation coefficient r=0.25, p=0.001), proteinuria (r=-0.21, p=0.005) and creatinine (r=-0.31, p<0.001). Furthermore, patients with a lower GFR were older, had a higher BMI and lower serum albumin concentrations than those with a higher GFR.

**Homoarginine and progression of CKD.** Of the 182 patients with a homoarginine measurement at baseline, 139 could be followed until the end of the study or occurrence of the primary renal endpoint. A total of 56 of the 139 patients (40.3 %) experienced a renal disease progression (doubling of baseline serum creatinine and/or terminal renal failure necessitating renal replacement therapy). Homoarginine concentrations were significantly lower in these patients as compared to those without kidney disease progression (2.19±0.93 versus 2.71±1.13 µmol/L, respectively, p=0.005). The further characteristics of the patients with and without kidney disease progression are presented in Table 2.

We performed Cox regression analyses considering the time of reaching the kidney endpoint (Table 3). The age and sex-adjusted hazard ratio to suffer a kidney endpoint was significantly higher by 62 % with each 1-standard deviation (1.1 µmol/L) decrease of homoarginine (HR 1.62, 95 % CI 1.16-2.27, p=0.005). Further adjustment for proteinuria did not change the results (HR 1.56, 95 % CI 1.11-2.20, p=0.01). Additional adjustment for measured GFR slightly attenuated the association, resulting in a borderline significant hazard ratio of 1.40 (95 % CI 0.98-1.98, p=0.06)

### DISCUSSION

This is the first study that describes plasma homoarginine concentrations in patients with primary non-diabetic chronic kidney disease not requiring dialysis. The main findings of this study are that 1) circulating homoarginine concentrations in CKD patients are significantly lower with lower GFR; 2) homoarginine concentrations were substantially lower in patients with kidney disease progression as compared to those without progression; 3) circulating homoarginine concentrations are inversely associated with the risk to reach a kidney endpoint, independently of age, sex and proteinuria. This association was slightly attenuated after additional adjustment for GFR (p=0.06).

Interestingly, up to now all parameters investigated in the MMKD Study showed a correlation of high concentrations with impaired kidney function as well as progression of CKD which points at a (direct or indirect) role of the kidney in their elimination (Kollerits et al., 2007, Kidney Int 71: 1279-1286; Kronenberg et al., 2000, J Am Soc Nephrol 11: 105-115; Fliser et al., 2007, J Am Soc Nephrol 18: 2600-2608). Homoarginine is the first parameter, which showed not only lower concentrations at decreased kidney function, but also a higher probability of CKD progression at low concentrations. This is in line with the fact that the kidney is probably the most important site of homoarginine production. The observed association with kidney function and CKD progression therefore likely reflects decreasing synthesis capacity of the kidney rather impairment of renal ultrafiltration.

Homoarginine is a cationic amino acid, which is derived from lysine. Homoarginine is formed from lysine in two independent synthesis routes. First, it is produced in a homologous urea cycle, in which ornithine is replaced by lysine (Ryan et al., 1969, Arch Biochem Biophys 131: 1285-1295; Ryan and Wells, 1964, Science 144: 1122-1127). The second synthesis route is a direct transaminidation reaction of lysine mainly located in the kidney and mediated by the l-arginine:glycine amidinotransferase (AGAT or GATM). This enzyme also catalyzes an essential step of the creatine metabolism, namely the conversion of arginine and glycine into guanidinoacetate and ornithine. This is a reversible process which depends on the concentration of co-reactants. If lysine is sufficiently available, AGAT converts lysine together with guanidinoacetate to form homoarginine (and glycine) (Ryan et al., 1969, Arch Biochem Biophys 131: 1285-1295). Since AGAT is able to catalyze a number of transamidination reactions, homoarginine can also be formed from other substrates, e.g. by the AGAT-mediated reaction of guanidinopropionic acid with ornithine. AGAT is mainly present in the kidney, but also in various other organs including the liver, the pancreas and the heart. The importance of the kidney for homoarginine synthesis is supported by our observation of a virtually linear association between homoarginine concentrations and GFR. In our cross-sectional analyses, we found homoarginine concentrations lower at lower levels of GFR. These findings are entirely consistent with genome-wide association studies (GWAS) showing that polymorphisms of AGAT are significantly associated with GFR (Chambers et al., 2010, Nat Genet 42: 373-375). It should also be stressed that homoarginine deficiency is not simply a marker of malnutrition because homoarginine is believed to be mainly derived from endogenous synthesis and not from nutrition. In this context, it is also important to note that neither BMI nor albumin were associated with the progression of kidney disease and that the relationship of homoarginine with progression was still significant after adjustments for albumin and BMI (HR 1.44, 95% CI 1.01-2.05, p=0.045).

Our finding of an association of homoarginine with kidney function is furthermore in line with previous clinical studies. In the LURIC cohort comprising patients undergoing coronary angiography, homoarginine was significantly correlated to GFR with a correlation coefficient of 0.23 (p<0.001). The mean homoarginine concentration in this cohort was 2.6±1.1 µmol/L and patients had a mean GFR of 81±19 ml/min (Maerz et al, 2010, Circulation 122:967-975). Of note, homoarginine concentrations were less than half as high in patients with end-stage renal disease requiring maintenance dialysis: patients in the 4D study had a mean homoarginine concentration of 1.2±0.5 µmol/l (Maerz et al, 2010, Circulation 122:967-975). The concentrations that identified the respective interquartile ranges in the two cohorts were meaningfully lower in the 4D as compared to the LURIC study (0.87-1.4µmol/L versus 1.85-3.1µmol/L). The interquartile range for homoarginine in the MMKD Study was 1.81-3.13 µmol/L, which is close to that found in the LURIC study. Patients in the present study had mild to moderate kidney failure and a mean GFR of 69±43 ml/min. The main explanation for the decreased homoarginine concentrations in patients with advanced stages of kidney disease might be due to reduced activity of AGAT (see above). This hypothesis is supported by an experimental study by Tofuku et al. who found a decreased renal AGAT activity in rabbits with chronic kidney failure (Tofuku et al, 1985, Nephron 41: 174-178). Similarly, plasma concentrations of homoarginine were significantly decreased in nephrectomized rats as compared to a sham-operated control group (Al Banchaabouchi et al., 2001, Metabolism 50: 1418-1425). Taken together, low homoarginine concentration may therefore be an early indicator of kidney failure and potentially useful as novel biomarker.

As described above, homoarginine can be formed within an alternative urea cycle. Through this cycle, homoarginine serves as a precursor of NO by increasing the intracellular concentration of L-arginine, which is the main substrate for NO synthase. Homoarginine is also an inhibitor of arginase, further increasing the bioavailability of arginine and enhancing nitric oxide formation. Thus, evidence suggests homoarginine to increase the availability of nitric oxide (NO), lack of which is associated with endothelial dysfunction and contributing to kidney damage. In previous studies, homoarginine was found inversely correlated to markers of impaired endothelial function, i.e. ICAM-1 and VCAM-1 (Maerz et al, 2010, Circulation 122:967-975). It may therefore be speculated whether lack of homoarginine is not only a risk marker, but potentially a risk factor in the progression of CKD. In our study, low homoarginine was significantly associated with disease progression, which was independent of age, sex and proteinuria and which was attenuated when adjusted for baseline GFR. However, adjustment for GFR could be considered as an overadjustment since homoarginine is significantly associated with GFR and may even mediate the effect of lower GFR on progression of kidney disease leading hypothetically to a vicious cycle.

The previously shown experimental effects of homoarginine on blood pressure regulation, insulin secretion and platelet aggregation may represent further pathways by which homoarginine could potentially affect the course of kidney function. Previous studies found that administration of L-homoarginine increased urinary excretion of nitrate, the degradation product of NO, and reduced blood pressure in salt-sensitive hypertensive rats (Chen and Sanders, 1993, Hypertension 22: 812-818). Implications of homoarginine concentrations on NO availability in humans, however, remain to be clarified. Furthermore, homoarginine is known to be an inhibitor of bone alkaline phosphatase (Kozlenkov et al., 2004, J Bone Miner Res 19: 1862-1872), thus potentially being important in the prevention of CKD-related complications such as bone and mineral disorders.

A strength of the study is that GFR was not calculated by a formula but was measured by iohexol clearance which is considered an exact method to measure kidney function. Potential limitations of our study deserve also comments. Due to the observational design of our study, we cannot prove causality of the associations between homoarginine, kidney function and kidney disease progression. Furthermore, our study is limited by the sample size, and homoarginine measurements were not available in all patients of the MMKD cohort. This might also be an explanation why the association of homoarginine with progression of CKD was only of borderline significance if adjusted for baseline GFR and proteinuria (p=0.06), reflecting a too small statistical power rather than pronounced confounding by GFR. The lack of material in 38 patients out of 177 followed did not produce a particular selection bias: these 38 patients without homoarginine measurement were not different in major risk factors compared to those with measurements available (data not shown).

In conclusion, we have found homoarginine concentrations directly correlated with kidney function in patients with CKD. Furthermore, low homoarginine concentrations were significantly associated with the progression of kidney disease. Low homoarginine concentrations may be an early indicator of kidney failure and potentially useful as a marker of disease progression. Whether homoarginine metabolism is causally relevant for kidney disease progression deserves further studies including randomized controlled trials with homoarginine supplementation.

**TABLE 1. Baseline clinical and laboratory data of 182 patients with non-diabetic chronic kidney disease stratified by GFR stages according to K/DOQI guidelines.**

| **Variable** | | GFR (mL/min/1.73 m²) | | | | |
|---|---|---|---|---|---|---|
| | all patients | ≥90 (n=59) | 60 - 89 (n=35) | 30 - 59 (n=51) | <30 (n=37) | p-value |
| Sex: males/females, n (%) | 122/60 (67.0/33.0) | 41/18 (69.5/30.5) | 24/11 (68.6/31.4 ) | 35/16 (68.6/31.4) | 22/15 (59.5/40.5) | 0.75 |
| Age (years) | 45.8±12.8 | 40.5±13.4 | 45.8±12.3 | 45.9±11.7 | 54.2±9.0 | <0.0 01 |
| BMI (kg/m²) | 25.21±3.6 | 24.2±3.3 | 25.8±3.6 | 25.1±3.1 | 26.2±4.3 | 0.04 |
| Current smokers, n (%) | 36(20) | 15(25) | 8(23) | 6(12) | 7(19) | 0.73 |
| Systolic blood pressure (mmHg) | 137±21 | 135±22 | 138±25 | 138±18 | 138±19 | 0.82 |
| Diastolic blood pressure (mmHg) | 86±13 | 83±13 | 86±13 | 86±13 | 88±14 | 0.40 |
| Serum albumin (g/dL) | 4.6±0.4 | 4.7±0.4 | 4.4±0.6 | 4.6±0.4 | 4.5±0.4 | 0.00 5 |
| Proteinuria (g/24h/1.73m²) | 0.90±0.90 (0.18;0.55;1.2 6) | 0.56±0.65 (0.12;.0.35;0. 73) | 1.10±1.11 (0.17;0.60 ; 1.80) | 1.01±0.95 (0.22;0.55;1. 78) | 1.10±0.81 (0.54;0.95; 1.52) | 0.00 1 |
| GFR (mL/min/1.73m²) | 69±43 (38;63;96) | 120±29 (96;111;134) | 73±9 (65;70;81) | 45±7 (40;44;50) | 19±8 (12;18;27) | <0.0 01 |
| Creatinine-standardized measurement (µmol/L) | 179±113 (96;135;231) | 89±21 (73;84;107) | 136±49 (108;127; 142) | 202±72 (154;188;23 7) | 334±115 253;319;4 22) | <0.0 01 |
| Homoarginine (µM/L) | 2.57±1.09 | 2.90±1.02 | 2.64±1.06 | 2.52±1.24 | 2.05±0.78 | 0.00 2 |

| | | | | | | |
|---|---|---|---|---|---|---|
| GFR denotes glomerular filtration rate measured by iohexol clearance, BMI; body-mass index. Data are presented as mean ± SD and 25^{th}, 50^{th} (median) and 75^{th} percentiles for skewed variables where appropriate. P-values are for comparison across all four groups obtained from Kruskal-Wallis test, one-way ANOVA and X² test where appropriate. | | | | | | |

**TABLE 2. Baseline clinical and laboratory data of the 139 patients who completed follow-up and stratified by patient groups with and without progression of chronic kidney disease.**

| **Variable** | **All patients (n=139)** | **Non-progressors (n=83)** | **Progressors (n= 56)** | ***P-*value*^{a}*** |
|---|---|---|---|---|
| Sex: males/females, n (%) | 90/49 (64.7/35.3) | 54/29 (65.1/34.9) | 36/20 (64.3/35.7) | 0.93 |
| Age (years) | 46.6±12.5 | 45.2±13.0 | 48.6±11.4 | 0.18 |
| BMI (kg/m²) | 25.3±3.6 | 25.0±3.5 | 25.7±3.8 | 0.22 |
| Current smokers, n (%) | 22 (15.8) | 11 (13.3) | 11 (19.6) | 0.32 |
| Systolic blood pressure (mmHg) | 136±20 | 135±22 | 138±17 | 0.32 |
| Diastolic blood pressure (mmHg) | 85±12 | 84±13 | 88±12 | 0.09 |
| Serum albumin (g/dL) | 4.6±0.4 | 4.6±0.5 | 4.6±0.4 | 0.99 |
| Proteinuria (g/24h/1.73 m²) | 1.00±0.92 (0.24;0.69;1.54) | 0.80±0.93 (0.14;0.36;1.14) | 1.30±0.84 (0.63;1.10;1.85) | <0.001 |
| GFR (mL/min/1.73m²) | 62±41 (34;52;87) | 79±41 (50;70;100) | 37±24 (19;33;45) | <0.001 |
| Creatinine (µmol/L) | 195±118 (105;157;253) | 131±63 (90;119;158) | 289±119 (194;281;385) | <0.001 |
| Homoarginine (µM/L) | 2.50±1.08 | 2.71±1.13 | 2.19±0.93 | 0.005 |

| | | | | |
|---|---|---|---|---|
| GFR denotes glomerular filtration rate measured by iohexol clearance, BMI; body-mass index. Data are presented as mean ± SD and 25^{th}, 50^{th} (median) and 75^{th} percentiles for skewed variables where appropriate. *^{a}* P value for comparison between progressors and non-progressors. | | | | |

**TABLE 3: The association of baseline variables with progression of kidney disease during the observation period using multiple Cox proportional hazards regression models.**

| | **Model 1** | | **Model 2*^{a}*** | | **Model 3***^{b,c}* | |
|---|---|---|---|---|---|---|
| | **Adjusted for age, sex** | | **Adjusted for age**, **sex, and proteinuria** | | **Adjusted for age, sex, GFR and proteinuria** | |
| Variable (1 SD decrement) | HR (95% CI) | p | HR (95% CI) | p | HR (95% CI) | p |

| **Entire patient group** | | | | | | |
|---|---|---|---|---|---|---|
| GFR (-41 mUmin/1.73 m²) | 5.05 (2.90-8.77) | <0.001 | 5.26 (2.94-9.43) | <0.001 | 5.26 (2.94-9.43) | <0.0 01 |
| Proteinuria (0.92 g/24h/1.73 m2) | 1.38(1.09-1.75) | 0.007 | 1.38 (1.09-1.75) | 0.007 | 1.37 (1.06-1.76) | 0.01 5 |
| Homoarginin e (-1.1 µM/L) | 1.62 (1.16-2.27) | 0.005 | 1.56(1.11-2.20) | 0.010 | 1.40 (0.98-1.98) | 0.06 |

| | | | | | | |
|---|---|---|---|---|---|---|
| *^{a}* The hazard ratio for proteinuria is only adjusted for age and sex and is therefore the same as model 1.*^{b}* The hazard ratio for GFR is only adjusted for age, sex and proteinuria and is therefore the same as model 2. *^{c}* The hazard ratio for proteinuria is only adjusted for age, sex and GFR. The hazard ratios (HR) and 95% confidence intervals (CI) were determined by univariate and multiple Cox proportional hazards regression analysis and are indicated for each decrement of 1 standard deviation (SD). For proteinuria, hazard ratios are indicated for each increment of 1 SD. | | | | | | |

### Example 2

### MATERIAL and METHODS

**Study Design and Participants.** The methodology of the ALERT study has previously been reported in detail (ref). Briefly, this was a prospective randomized controlled trial investigating the effect of fluvastatin, 40-80 mg daily, on cardiac and renal outcomes in renal transplant recipients over a follow-up period of 5-6 years. The study included 2102 renal transplant recipients, aged 30 - 75 years, who had received a renal transplant more than 6 months before and had a serum cholesterol concentration between 4.0 and 9.0 mmol/L (155-348 mg/dL). Exclusion criteria were statin therapy, familial hypercholesterolemia, a life expectancy of less than one year, or if patients experienced an acute rejection within the last 3 months before randomization. Study visits took place at randomization, at 6 weeks after randomization and every six months thereafter until the date of death, censoring, or end of the study. At each follow-up, blood samples were taken and clinical information including any adverse events was recorded. The study conformed with the principles outlined in the Declaration of Helsinki and adhered to the International Conference on Harmonisation guidelines for Good Clinical Practice. It was approved by the medical ethical committee of each participating centre, and all patients gave their written informed consent before inclusion.

**Laboratory measurements.** Homoarginine was measured in blood samples taken at baseline and stored at -80°C, using a reverse-phase HPLC method. Within-day coefficients of variation (CV) were 4.7% (1.21 µM) and 2.2% (3.53 µM), and between-day CV were 7.9% (1.25 µM) and 6.8% (3.66 µM), respectively. All blood samples were taken in the morning before the administration of medication. The measurements of homoarginine were performed at the Department of Clinical Chemistry at the Medical University of Graz, Austria. Furthermore, measurements of fasting lipids, serum creatinine, creatine kinase, and hepatic enzymes were performed centrally at Medinet laboratory in Breda, the Netherlands.

**Outcome assessment.** The primary endpoint of the ALERT study was defined as a composite of death from cardiac causes, nonfatal myocardial infarction (MI), or coronary revascularisation procedure, whichever occurred first (major adverse cardiovascular event; MACE). Coronary revascularisation procedures included coronary artery bypass grafting or percutaneous coronary interventions. An adjudicated MI was classified as definite if a new Q-wave developed in the presence of abnormal cardiac markers or symptoms, or pathological ST elevations and T-wave changes developed in the presence of abnormal cardiac markers plus symptoms. An MI was classified as probable if pathological ST elevations and T-wave changes developed in the presence of abnormal cardiac markers or symptoms. Predefined secondary endpoints were the individual cardiac events, combined cardiac death or non-fatal MI (CDNFMI), combined cerebrovascular events (CBV), non-cardiovascular death, all-cause mortality, and the composite renal endpoint of graft loss or doubling of serum creatinine (GFDSC). The ALERT Study endpoints were centrally adjudicated by four members of the endpoint committee blinded to study treatment and according to pre-defined criteria. For the present analysis, primary endpoint of MACE, combined cardiac death or non-fatal MI (CDNFMI), combined cerebrovascular events (CBV), non-cardiovascular death, all-cause mortality, and the composite renal endpoint of graft loss or doubling of serum creatinine (GFDSC), were all chosen as separate outcome measures. The categorization of these events was based on the primary judgement of the endpoint committee during the ALERT Study.

**Statistical Analysis.** Continuous variables were expressed as mean with standard deviation or median with interquartile range (IQR) as appropriate, and categorical variables were expressed as percentages. In the initial analysis, the treatment and placebo arms were analyzed separately for clinical events. As the two arms showed no significant heterogeneity in relationships between homoarginine as a risk factor and event outcome, subsequent analysis was performed on the pooled patient population. The study population was divided into four groups, according to quartiles of homoarginine levels at baseline: ≤1.39 µmol/L, >1.39 to ≤1.81 µmol/L, >1.81 to ≤ 2.33 µmol/L, >2.33 µmol/L. Demographic and clinical baseline characteristics were compared using independent samples t-test and w2-test for continuous and categorical variables respectively. We assessed the association of baseline homoarginine with the specific cardiovascular and renal events: primary endpoint of MACE, combined cardiac death or non-fatal MI (CDNFMI), combined cerebrovascular events (CBV), non-cardiovascular death, all-cause mortality, and the composite renal endpoint of graft loss or doubling of serum creatinine (GFDSC). In the categorical analyses, patients of the highest homoarginine quartile were used as the reference group. Kaplan-Meier curves were performed in each group and the log rank test was computed to compare the curves. Relative risks were determined by Cox regression analyses, i.e. hazard ratios (HRs) and corresponding 95 % confidence intervals. The Cox regression analyses were adjusted for the cofounders age, sex, fluvastatin treatment, diabetes mellitus, CAD, smoking status, systolic blood pressure, LDL-cholesterol, and estimated GFR. All p-values are reported two-sided. Analyses were performed using SPSS version 19.0.

### RESULTS

**Patient characteristics.** Of all 2102 patients included into the ALERT study, 1870 patients had a homoarginine measurement at baseline. The mean duration of follow-up was 6.7 years. The mean (standard deviation) homoarginine concentration at baseline was 1.95 (0.85) µmol/L; with no significant difference between the fluvastatin and placebo groups. The baseline patient characteristics are shown in Table 1. Patients with low homoarginine concentration were more likely smokers and had a higher burden of diabetes; furthermore the percentage of female patients was higher. Low homoarginine concentrations were associated with a lower BMI, lower estimated GFR, higher creatinine, CRP and phosphate levels. Age, lipid profile, blood pressure, the presence of hypertension and coronary artey disease was comparable across homoarginine concentrations.

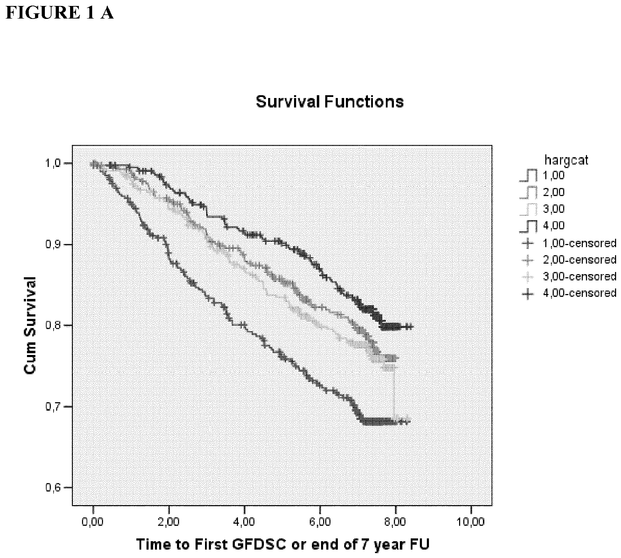

**Homoarginine and decline of renal function.** Of all patients, a total of 370 patients reached the composite renal endpoint of graft loss or doubling of serum creatinine (GFDSC) during follow-up. Homoarginine concentrations were significantly lower in the patients with renal disease progression as compared to those without (1.87 vs 2.01 µmol/L, respectively, p<0.05). We performed Kaplan-Meier and Cox regression analyses considering the time to reaching the renal endpoint (Figure 1A and Table 2). For patients of the lowest homoarginine quartile, the unadjusted hazard to achieve the renal endpoint was significantly 2 fold increased as compared to patients of the highest homoarginine quartile (HR 1.97, 95% CI 1.47-2.64). The association mainly persisted after adjustment for confounders including age, sex, diabetes mellitus, CAD, smoking status, systolic blood pressure, LDL-cholesterol, and baseline GFR (HR 1.58, 95% CI 1.15-2.16).

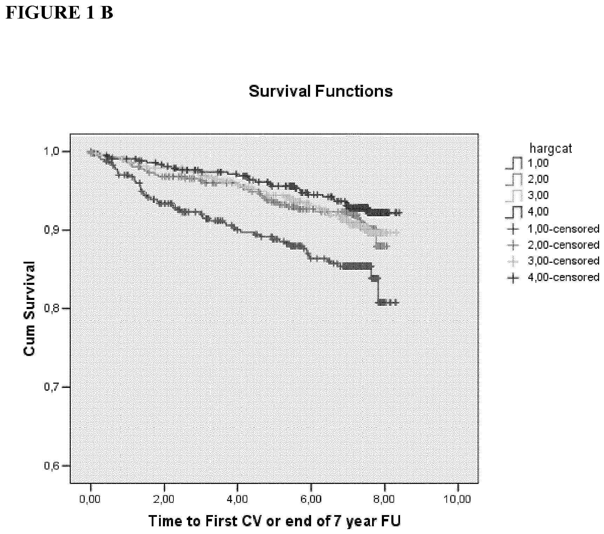

**Homoarginine and the risk of cerebrovascular events.** Lower homoarginine levels at baseline were associated with higher incidences of cerebrovascular events. Of all 157 cerebrovascular events, 55 occurred in patients of the lowest homoarginine quartile, 35 in patients of the second quartile, 38 in those of the third quartile and 29 in patients of the highest homoarginine quartile. By Cox regression analyses, the crude risk of cerebrovascular events significantly increased more than 2fold in patients of the lowest as compared to patients of the highest homoarginine quartile (HR crude 2.29, 95% CI 1.46-3.59, Table 2). The association persisted after adjustment for confounders (HR 2.38, 95% CI 1.47-3.87). Results of the Kaplan-Meier analyses are shown in Figure 1B.

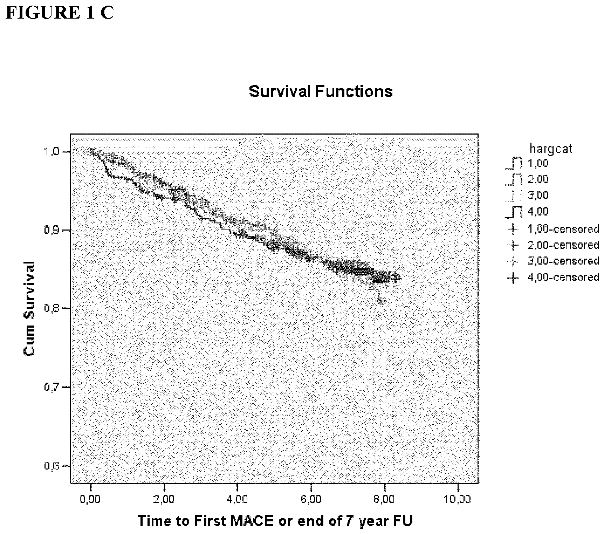

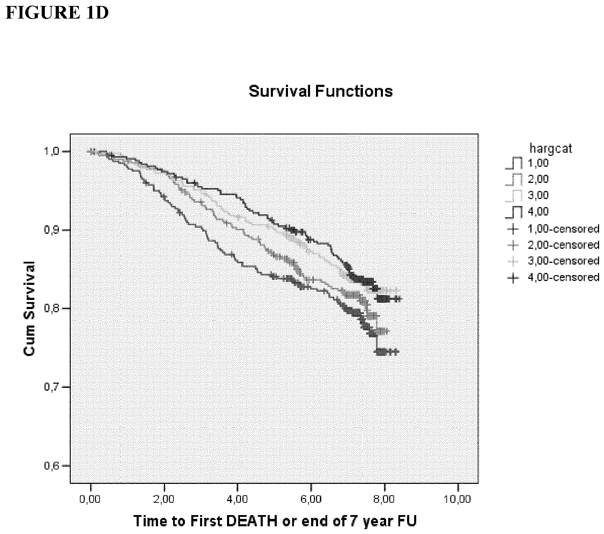

**Homoarginine and the risk of cardiovascular events, non-cardiovascular and all-cause mortality.** In contrast to the results seen for cerebrovascular events, homoarginine did not meaningfully affect the risk of cardiac death or non-fatal myocardial infarction (CDNFMI, Table 2). Similarly, the endpoint of major adverse cardiovascular events was not affected (Figure 1C). There was a tendency for an increased risk of non-cardiovascular mortality. Patients of the lowest homoarginine quartile exhibited an adjusted 44 % increased risk as compared to patients of the highest quartile; this association however was not significant (HR 1.44, 95 % CI 0.89-2.33). The incidence of all-cause mortality significantly increased with low homoarginine concentrations in cruse analyses (HR 1^{st} versus 4^{th} quartile 1.43, 95%CI 1.04-1.96); this association was slightly attenuated after adjustment for confounders (HR 1.39, 95%CI 0.98-1.96). Results of the Kaplan-Meier analyses for all-cause mortality are shown in Figure 1D.

**TABLE 1: Patient characteristics according to quartiles of homoarginine at baseline; study population n=1870**

| **Characteristic** | **homoarginine (µmol/L)** | | | |
|---|---|---|---|---|
| | <1.39 | 1.39-1.81 | 1.81-2.33 | >2.33 |
| | n=471 | n=467 | n=470 | n=462 |
| Age (years) | 48 ± 10 | 50 ± 11 | 50 ± 11 | 50 ± 11 |
| Gender (%men) | 52 | 61 | 72 | 77 |
| Smoker (%) | 25 | 20 | 16 | 14 |
| Diabetes mellitus (%) | 26 | 20 | 17 | 15 |
| Systolic BP (mmHg) | 144 ± 19 | 144 ± 20 | 145 ± 19 | 145 ± 18 |
| BMI (kg/m²) | 24.7 ± 4.2 | 25.4 ± 4.4 | 26.3 ± 4.4 | 26.5 ± 4.2 |
| CAD (%) | 9 | 10 | 8 | 11 |
| Hypertension (%) | 74 | 75 | 71 | 75 |

| Transplant characteristics | | | | |
|---|---|---|---|---|
| donor age (years) | 42 ± 15 | 42 ± 16 | 40 ± 16 | 41 ± 15 |
| total time on RRT (years) | 7.6 ± 5.1 | 7.5 ± 5.2 | 7.5 ± 4.8 | 7.0 ± 4.5 |
| cold ischemia time (h) | 19 ±8 | 20 ± 7 | 19 ±7 | 19 ±8 |
| Laboratory parameters C-reactive protein (mg/L) | 4.3 ±7.4 | 4.2 ±7.6 | 3.2 ±5.3 | 3.4 ±6.4 |
| Total cholesterol (mmol/L) | 6.5± 1.2 | 6.4 ± 1.1 | 6.5 ± 1.1 | 6.5 ± 1.2 |
| LDL cholesterol (mmol/L) | 4.1 ± 1.1 | 4.1 ± 1.0 | 4.2 ± 1.0 | 4.2 ± 1.1 |
| HDL cholesterol (mmol/L) | 1.4 ± 0.5 | 1.4 ±0.5 | 1.3 ±0.4 | 1.3 ±0.5 |
| Creatinine (µmol/l) | 155 ± 63 | 145 ± 54 | 143 ± 53 | 140 ± 46 |
| estimated GFR | 45 ± 16 | 48 ± 17 | 50 ± 15 | 51 ± 17 |
| Calcium (mmol/L) | 2.4 ± 0.2 | 2.4 ± 0.2 | 2.4 ± 0.2 | 2.4 ± 0.1 |
| Phosphate (mg/dL) | 3.8 ± 0.8 | 3.6 ± 0.7 | 3.6 ± 0.7 | 3.5 ± 0.6 |
| Primary renal disease | | | | |
| Glomerulonephritis | 30 | 31 | 39 | 43 |
| PCKD | 16 | 17 | 15 | 15 |
| Diabetic nephropathy | 21 | 16 | 10 | 8 |
| Pyelo-interstitial | | | | |
| nephritis | 15 | 14 | 13 | 10 |
| Hypertension | 6 | 4 | 4 | 6 |
| medication | | | | |
| ACE- inhibitors (%) | 50 | 51 | 51 | 52 |
| Beta-blockers (%) | 63 | 63 | 59 | 63 |
| Diuretics (%) | 59 | 57 | 55 | 57 |
| active vitamin D | 22 | 19 | 18 | 11 |

| | | | | |
|---|---|---|---|---|
| Values are presented as means (SD) or median (interquartile range) or %. Abbreviations: BMI, body mass index; BP, blood pressure; CAD, coronary artery disease. | | | | |

**Table 2: Hazard ratios with 95% confidence intervals (HR, 95% CI) for study outcomes within the homoarginine quartiles compared with the fourth quartile**

| Outcome | | homoarginine quartiles Q1 n=725 | Q2 n=149 | Q3 n=157 | Q4 n=149 |
|---|---|---|---|---|---|
| | | | | | |

| ***cerebrovascular events*** | | | | | |
|---|---|---|---|---|---|
| | Nr of events | 55 (13.8%) | 35 (8.6%) | 38 (8.9%) | 29 (6.9%) |
| | Crude HR (95% CI) | 2,29 (1.46-3.59) | 1.35 (0.82-2.20) | 1.34 (0.82-2.17) | 1 |
| | Adj. HR (95% CI) | 2.38 (1.47-3.87) | 1.31 (0.79-2.19) | 1.29 (0.78-2.12) | 1 |

| ***MACE*** | | | | | |
|---|---|---|---|---|---|
| | Nr of events | 56 (140%) | 57 (13.9%) | 66 (15.4%) | 63 (14.9%) |
| | Crude HR (95% CI) | 1.00 (0.70-1.43) | 0.92 (0.64-1.34) | 1.03 (0.73-1.46) | 1 |
| | Adj. HR (95% CI) | 0.91 (0.61-1.33) | 0.91 (0.62-1.32) | 1.00 (0.70-1.43) | 1 |

| ***CDNFMI*** | | | | | |
|---|---|---|---|---|---|
| | Nr of events | 41 (10.3%) | 41 (10.0%) | 49 (11.4%) | 47 (111 %) |
| | Crude HR (95% CI) | 0.98 (0.64-1.49) | 0.95 (0.62-1.44) | 1.04 (0.70-1.55) | 1 |
| | Adj. HR (95% CI) | 0.86 (0.55-1.36) | 0.85 (0.55-1.33) | 1.00 (0.67-1.51) | 1 |

| ***NCVDTH*** | | | | | |
|---|---|---|---|---|---|
| | Nr of events | 43 (10.8%) | 40 (9.8%) | 35 (8.2%) | 37 (8.7%) |
| | Crude HR (95% CI) | 1.35 (0.87-2.10) | 1.21 (0.77-1.89) | 0.96 (0.61-1.53) | 1 |
| | Adj. HR (95% CI) | 1.44 (0.89-2.33) | 1.27 (0.80-2.01) | 0.93 (0.58-1.50) | 1 |

| *A**ll-cause mortality*** | | | | | |
|---|---|---|---|---|---|
| | Nr of events | 86 (21.5%) | 78 (19.1 %) | 71 (16.6%) | 70 (16.5%) |
| | Crude HR (95% CI) | 1.43 (1.04-1.96) | 1.25 (0.90-1.72) | 1.03 (0.74-1.43) | 1 |
| | Adj. HR (95% CI) | 1.39 (0.98-1.96) | 1.23 (0.88-1.72) | 1.01 (0.72-1.42) | 1 |

| ***GFDSC*** | | | | | |
|---|---|---|---|---|---|
| | Nr of events | 116 (29.0%) | 83 (20.3%) | 97 (22.6%) | 74 (17.5%) |
| | Crude HR (95% CI) | 1.97 (1.47-2.64) | 1.26 (0.92-1.73) | 1.38 (1.02-1.86) | 1 |
| | Adj. HR (95% CI) | 1.58 (1.15-2.16) | 1.06 (0.77-1.48) | 1.46 (1.07-1.99) | 1 |

| | | | | | |
|---|---|---|---|---|---|
| adjustments were made for: age, sex, diabetes mellitus, smoking status, systolic blood pressure, LDL-cholesterol, coronary artery disease, eGFR. MACE: major adverse cardiovascular events; CDNFMI: cardiac death or non-fatal myocardial infarction NCVDTH: non-cardiovascular mortality; GFDSC: graft failure or doubling of serum creatinine | | | | | |

### Figure Legends

Figure 1 A-D: Kaplan Meier curves for the time to A) graft failure or doubling of serum creatinine, B) cerebrovascular events C) major adverse cardiac events, D) all-cause mortality in subgroups of patients according to the homoarginine concentrations at baseline (quartiles)

## Claims

1. A in vitro method of determining the progression of chronic kidney disease (CKD) and/or kidney transplant failure in a patient, wherein the method comprises the steps of
a) determining the amount of homoarginine in a sample of the patient;
b) comparing the determined amount of homoarginine with a reference amount, and
c) determining the progression of chronic kidney disease (CKD) and/or kidney transplant failure, wherein determining the progression of chronic kidney disease (CKD) refers to the assessment of kidney function in a patient, wherein
the method further comprises determining the risk of chronic kidney disease progression and/or the risk of kidney transplant failure in a patient, wherein determining the risk of chronic kidney disease progression or the risk of kidney transplant failure refers to assessing the probability according to which a subject will suffer from a progressed disease status with a certain time window,
d) wherein a decreased amount of homoarginine in comparison to the reference amount indicates progression of chronic kidney disease (CKD) and/or kidney transplant failure, or an increased risk of progression of chronic kidney disease (CKD) and/or increased risk of kidney transplant failure within a certain time window.

2. The method of claim 1, wherein the progression of chronic kidney disease (CKD) and/or the risk of kidney transplant failure is the higher, the lower the amount of homoarginine is in the patient's sample.

3. The method of claim 1 or claim 2, wherein the decreased amount of homoarginine correlates with a decreased renal function, preferably with a decreased glomerular filtration rate (GFR) and/or an increased level of serum creatinine in the patient.

4. The method of any claims 1 to 3, wherein the progression of chronic kidney disease (CKD) in a patient includes end-stage renal disease.

5. The method of any claims 1 to 4, wherein the sample is a blood sample, preferably a plasma sample.

6. The method of any claims 1 to 5, wherein the amount of homoarginine in step a) is determined by means of reverse-phase high-performance liquid chromatography (HPLC).

7. In vitro use of homoarginine as a marker in kidney transplant failure.

## Patentansprüche

1. Verfahren zur Bestimmung in vitro des Fortschreitens einer chronischen Nierenerkrankung (CKD) und/oder eines Nierentransplantatversagens bei einem Patienten, wobei das Verfahren die folgenden Schritte umfasst:-
a) Bestimmung der Menge an Homoarginin in einer Probe des Patienten;
b) Vergleichen der ermittelten Menge an Homoarginin mit einer Referenzmenge, und
c) Bestimmen des Fortschreitens einer chronischen Nierenerkrankung (CKD) und/oder eines Nierentransplantationsversagens, wobei sich das Bestimmen des Fortschreitens einer chronischen Nierenerkrankung (CKD) auf die Beurteilung der Nierenfunktion bei einem Patienten bezieht, wobei das Verfahren ferner das Bestimmen des Risikos des Fortschreitens einer chronischen Nierenerkrankung und/oder des Risikos eines Nierentransplantationsversagens bei einem Patienten umfasst, wobei sich das Bestimmen des Risikos des Fortschreitens einer chronischen Nierenerkrankung oder des Risikos eines Nierentransplantationsversagens auf das Beurteilen der Wahrscheinlichkeit bezieht, nach der ein Subjekt innerhalb eines bestimmten Zeitfensters an einem fortgeschrittenen Krankheitszustand leiden wird,
d) wobei eine verringerte Menge an Homoarginin im Vergleich zur Referenzmenge ein Fortschreiten einer chronischen Nierenerkrankung (CKD) und/oder ein Nierentransplantationsversagen oder ein erhöhtes Risiko eines Fortschreitens einer chronischen Nierenerkrankung (CKD) und/oder ein erhöhtes Risiko eines Nierentransplantationsversagens innerhalb eines bestimmten Zeitfensters anzeigt.

2. Verfahren nach Anspruch 1, wobei das Fortschreiten der chronischen Nierenerkrankung (CKD) und/oder das Risiko eines Nierentransplantationsversagens umso höher ist, je geringer die Menge an Homoarginin in der Probe des Patienten ist.

3. Verfahren nach Anspruch 1 oder Anspruch 2, wobei eine verringerte Menge an Homoarginin mit einer verringerten Nierenfunktion, insbesondere mit einer geringeren glomerulären Filtrationsrate (GFR) und/oder einem erhöhten Serumkreatininwert des Patienten korreliert.

4. Verfahren nach einem der Ansprüche 1 bis 3, wobei das Fortschreiten der chronischen Nierenerkrankung (CKD) bei einem Patienten die Nierenerkrankung im Endstadium einschließt.

5. Verfahren nach einem der Ansprüche 1 bis 4, wobei die Probe eine Blutprobe, vorzugsweise eine Plasmaprobe, ist.

6. Verfahren nach einem der Ansprüche 1 bis 5, wobei die Menge an Homoarginin in Schritt a) mittels Umkehrphasen-Hochleistungsflüssigkeitschromatographie (HPLC) bestimmt wird.

7. In vitro-Verwendung von Homoarginin als Marker bei Nierentransplantationsversagen.

## Revendications

1. Méthode in vitro de détermination de la progression d'une maladie rénale chronique (MRC) et/ou d'un échec de transplantation rénale chez un patient, dans laquelle la méthode comprend les étapes suivantes
a) déterminer la quantité d'homoarginine dans un échantillon du patient ;
b) comparer la quantité déterminée d'homoarginine avec une quantité de référence, et
c) déterminer la progression de la maladie rénale chronique (MRC) et/ou d'échec de la transplantation rénale, dans lequel la détermination de la progression de la maladie rénale chronique (MRC) se réfère à l'évaluation de la fonction rénale dans un patient, dans lequel la méthode comprend en outre la détermination du risque de progression de la maladie rénale chronique et/ou du risque d'échec de la transplantation rénale dans un patient, dans lequel la détermination du risque de progression de la maladie rénale chronique ou du risque d'échec de la transplantation rénale se réfère à l'évaluation de la probabilité selon laquelle un patient souffrira d'un état pathologique progressif dans une certaine fenêtre de temps,
d) dans lequel une quantité diminuée d'homoarginine par rapport à la quantité de référence indique une progression de la maladie rénale chronique (CKD) et/ou une insuffisance de transplantation rénale, ou un risque accru de progression de la maladie rénale chronique (CKD) et/ou un risque accru d'insuffisance de transplantation rénale dans une certaine fenêtre de temps.

2. Méthode de la revendication 1, dans laquelle la progression de la maladie rénale chronique (CKD) et/ou le risque d'échec de la transplantation rénale est d'autant plus élevé que la quantité d'homoarginine est basse dans l'échantillon du patient.

3. Méthode selon la revendication 1 ou la revendication 2, dans laquelle la quantité réduite d'homoarginine est corrélée avec une fonction rénale réduite, de manière préférentielle avec un taux de filtration glomérulaire (GFR) réduit et/ou un niveau accru de créatinine sérique dans le patient.

4. Méthode de l'une quelconque des revendications 1 à 3, dans laquelle la progression de la maladie rénale chronique (MRC) dans un patient comprend une maladie rénale au stade terminal.

5. La méthode de l'une quelconque des revendications 1 à 4, dans laquelle l'échantillon est un échantillon de sang, de préférence un échantillon de plasma.

6. La méthode de l'une quelconque des revendications 1 à 5, dans laquelle la quantité d'homoarginine à l'étape a) est déterminée au moyen d'une chromatographie liquide haute performance (HPLC) en phase inverse.

7. Utilisation in vitro de l'homoarginine comme marqueur de l'échec de la transplantation rénale.
